# EUROPÄISCHE PATENTSCHRIFT

(11) **EP 2 714 761 B1**
(45) Veröffentlichungstag und Bekanntmachung des Hinweises auf die Patenterteilung: **13.05.2015**
(21) Anmeldenummer: 12721564.8
(22) Anmeldetag: 22.05.2012
(51) Int. Cl.: C08G 18/12, C08G 18/28, C08G 18/44, C08G 18/66, C08G 18/75, C09D 175/04, A61L 33/06, C08J 3/07

(54) **HYDROPHILER SCHICHTVERBUND FÜR MEDIZINISCHE GERÄTE**
HYDROPHILIC LAYER COMPOSITE FOR MEDICAL EQUIPMENT
COMPOSITE STRATIFIÉ HYDROPHILE POUR APPAREILS MÉDICAUX

(30) Priorität: 24.05.2011 EP 11167262
(43) Veröffentlichungstag der Anmeldung: 09.04.2014
(73) Patentinhaber: Bayer Intellectual Property GmbH, 40789 Monheim (DE)
(72) Erfinder: KÖCHER, Jürgen, 40764 Langenfeld (DE)
(74) Vertreter: BIP Patents
(86) Internationale Anmeldenummer: PCT/EP2012/059464
(87) Internationale Veröffentlichungsnummer: WO 2012/160053

(56) Entgegenhaltungen:
- EP-A1- 1 721 947
- WO-A1-98/11854
- WO-A1-99/44668
- WO-A1-2008/101003
- WO-A1-2009/115263

## Beschreibung

Die vorliegende Erfindung betrifft einen Schichtverbund umfassend eine Haftvermittlerschicht und einen damit verbundenen hydrophilen Topcoat. Ein weiterer Gegenstand der Erfindung ist ein Verfahren zur Herstellung des Schichtverbundes.

Zur Beschichtung von innerten Substraten wie Metall oder Kunststoff werden heute häufig polymere Beschichtungsmaterialien eingesetzt. Allerdings ist die Haftung der Polymere zu diesen Substraten in vielen Fällen nicht ausreichend, so dass es bereits bei leichter mechanischer oder chemischer Belastung zu einem zumindest partiellen Ablösen der Beschichtung kommen kann. Daher werden heute bei der Lackierung von innerten Oberflächen mit polymeren Beschichtungsmaterialien häufig Mehrschichtsysteme verwendet. Diese umfassen eine Haftvermittlerschicht, die zuerst auf die Substratoberfläche aufgebracht wird. Auf die Haftvermittlerschicht wird dann erst das polymere Beschichtungsmaterial als Funktionsschicht appliziert. Um einen sicheren Halt des Beschichtungssystems zu gewährleisten, muss das Material der Haftvermittlerschicht so gewählt werden, dass es sowohl auf der Substratoberfläche gut haftet als auch eine starke Haftung zu der darüberliegenden polymeren Funktionsschicht aufweist.

Medizinische Geräte wie beispielsweise Katheter, Führungsdrähte oder Filtermaterialien werden häufig mit einer funktionalen Beschichtung versehen. Dabei werden Materialien verwendet, die die Oberflächen hydrophil und reibungsvermindernd ausrüsten. Im Kontakt mit Gewebe oder Körperflüssigkeiten werden diese beschichteten Geräteoberflächen dann leichter benetzt, so dass ihre Verwendung aufgrund der damit einhergehenden geringeren Reibung für den Patienten schmerzloser ist.

Allerdings kommt besonders bei medizinischen Geräten der Haftung der Beschichtungen auf den Geräteoberflächen ein besondere Bedeutung zu, da beispielsweise bei einem Katheter das ungewollte oder verfrühte Ablösen einer Beschichtung während eines medizinischen Eingriffs zu erheblichen Komplikationen führen kann.

Viele der heute für medizinische Geräte verwendeten Polymere sind strukturell so aufgebaut, dass sie leicht Wasser an der Oberfläche adsorbieren oder sogar in ihre Polymerstruktur aufnehmen können. Durch diese Wasseraufnahme kommt es oft erst zu dem gewünschten Effekt der verminderten Reibung. Gleichzeitig nimmt jedoch die Stärke der Haftung der Beschichtung durch die Wechselwirkung mit Wasser häufig dramatisch ab. Es ist daher bei medizinischen Geräten von besonderer Bedeutung, zusätzlich eine Haftvermittlerschicht vorzusehen, um die Gefahr der Ablösung der funktionalen Beschichtung zu verhindern.

In den Patentanmeldungen WO 2009/115263, WO 2009/115264, WO 2010/025840 und WO 2010/025841 sind wässrige Polyurethanharnstoffdispersionen und organische Polyurethanharnstofflösungen beschrieben, die sich besonders gut zur hydrophilen Ausrüstung medizintechnischer Geräteoberflächen eignen. Diese Materialien sind von ihrer Struktur her jedoch so beschaffen, dass sie besonders leicht mit Wasser in Wechselwirkung treten. Dies führt bei längerem Wasserkontakt dazu, dass die Haftung von Beschichtungen aus diesen Materialien auf metallischen oder polymeren Oberflächen teilweise oder sogar ganz verloren gehen kann.

Das Dokument WO 99/44668 offenbart einen eine geringe Reibung aufweisenden Leitdraht zur Positionierung eines medizinischen Instruments, der aus einer Grundierungsbeschichtung und einer hydrophilen, schmierigen Beschichtung, die über der Grundierungsbeschichtung angeordnet ist, ausgestattet ist. Die hydrophile, schwierige Beschichtung ist ein querverbundenes Polymer aus der Gruppe von Polyurethan, Polyharnstoff und Polyurethanharnstoff, das mit einer hydrophilen Komplex-Spezies komplexiert ist.

Die Aufgabe der vorliegenden Erfindung war es daher, einen Schichtverbund mit einer hydrophilen Oberfläche bereit zu stellen, der insbesondere auf Substraten wie Metall oder Kunststoff eine starke Haftung aufweist.

Diese Aufgabe ist durch einen Schichtverbund umfassend eine Haftvermittlerschicht und einen damit verbundenen hydrophilen Topcoat, wobei die Haftvermittlerschicht einen Polyurethanhamstoffumfasst, der durch Umsetzung wenigstens folgender Komponenten erhältlich ist:
a) einem Makropolyol;
b) einem Polyisocyanat;
c) einem Amin mit wenigstens zwei Isocyanat-reaktiven Gruppen;
d) einem monofimktionellen Polyoxyalkylenether; und
e) einer Carbonsäure mit wenigstens zwei Isocyanat-reaktiven Gruppen,
der hydrophile Topcoat einen Polyurethanharnstoff umfasst, der durch Umsetzung wenigstens folgender Komponenten erhältlich ist:
f) einem Makropolyol;
g) einem Polyisocyanat;
h) einem Amin mit wenigstens zwei Isocyanat-reaktiven Gruppen; und
i) einem monofunktionellen Polyoxyalkylenether
und der Polyurethanharnstoff des hydrophilen Topcoats keine Carbonsäuregruppen aufweist, gelöst.

So wurde überraschenderweise gefunden, dass bei dem erfindungsgemäßen Schichtverbund die Haftvermittlerschicht gleichzeitig eine besonders starke Haftung insbesondere zu Substraten aus Metall oder Kunststoff und zu dem hydrophilen Topcoat aufweist. Damit eignet sich der Schichtverbund besonders gut dazu, derartige Substarte dauerhaft und belastbar mit einer hydrophilen Beschichtung zu versehen.

Polyurethanharnstoffe im Sinne der vorliegenden Erfindung sind polymere Verbindungen, die mindestens zwei Urethangruppen enthaltende Wiederholungseinheiten der folgenden allgemeinen Struktur 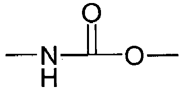 und mindestens eine Harnstoffgruppen enthaltende Wiederholungseinheit 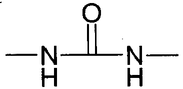 aufweisen.

Die erfindungsgemäß in der Haftvermittlerschicht und in dem Topcoat enthaltenen Polyurethanharnstoffe sind bevorzugt im Wesentlichen lineare Moleküle, können jedoch auch verzweigt sein, was jedoch weniger bevorzugt ist.

Das zahlenmittlere Molekulargewicht der Polyurethanharnstoffe beträgt bevorzugt 1000 bis 200000 und besonders bevorzugt von 5000 bis 100000, wobei zahlenmittlere Molekulargewicht gegen Polystyrol als Standard in Dimethylacetamid bei 30 °C gemessen wird.

Gemäß einer ersten bevorzugten Ausführungsform des erfindungsgemäßen Schichtverbunds ist vorgesehen, dass das Makropolyol a) und / oder das Makropolyol f) eine oder mehrere Verbindungen ausgewählt aus der Gruppe Polyesterpolyol, Polyetherpolyol, Polycarbonatpolyol umfasst.

Geeignete Polyetherpolyole sind solche Verbindungen, die durch Polymerisation von cyclischen Ethern wie Ethylenoxid, Propylenoxid, Butylenoxid, Tetrahydrofuran, Styroloxid oder Epichlorhydrin mit sich selbst, z. B. in Gegenwart von BF₃ oder basischen Katalysatoren, oder durch Anlagerung dieser Ringverbindungen, gegebenenfalls im Gemisch oder nacheinander, an Startkomponenten mit reaktionsfähigen Wasserstoffatomen wie Alkoholen und Aminen oder Aminoalkoholen, z. B. Wasser, Ethylenglykol, Propylenglykol-1,2 oder Propylenglykol-1,3, hergestellt werden können.

Bevorzugte hydroxylgruppenhaltige Polyether sind Verbindungen auf Basis von Ethylenoxid, Propylenoxid oder Tetrahydrofuran oder Mischungen dieser cyclischen Ether. Ganz besonders bevorzugte hydroxylgruppenhaltige Polyether basieren auf polymerisiertem Tetrahydrofuran. Es können noch weitere hydroxylgruppenhaltige Polyether zum Beispiel Verbindungen auf Basis Ethylenoxid oder Propylenoxid zugegeben werden, wobei dann aber vorzugsweise die Polyether auf Basis von Tetrahydrofuran zu mindestens 50 Gew.-% enthalten sind.

Als Hydroxylgruppen aufweisende Polycarbonate kommen Polycarbonate des durch OH-Zahl bestimmten Molekulargewichts von vorzugsweise 400 bis 6000 g/mol, besonders bevorzugt von 500 bis 5000 g/mol und ganz besonders bevorzugt von 600 bis 3000 g/mol in Frage, die beispielsweise durch Reaktion von Kohlensäurederivaten, wie Diphenylcarbonat, Dimethylcarbonat oder Phosgen, mit Polyolen, vorzugsweise Diolen, erhältlich sind. Als Diole eignen sich beispielsweise Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentan-1,3-diol, Di-, Tri- oder Tetraethylenglykol, Dipropylenglykol, Polypropylenglykole, Dibutylenglykol, Polybutylenglykole, Bisphenol A, 3(4),8(9)-Bis(hydroxymethyl)-tricyclo(5.2.1.0/2.6)decan , Tetrabrombisphenol A aber auch Lacton-modifizierte Diole. Bevorzugt enthält die Diolkomponente 40 bis 100 Gew.-% Hexandiol, insbesondere 1,6-Hexandiol und / oder Hexandiol-Derivate, vorzugsweise solche, die neben endständigen OH-Gruppen Ether- oder Estergruppen aufweisen, z. B. Produkte, die durch Umsetzung von 1 Mol Hexandiol mit mindestens 1 Mol, bevorzugt 1 bis 2 Mol Caprolacton oder durch Veretherung von Hexandiol mit sich selbst zum Di- oder Trihexylenglykol erhalten werden können. Auch Polyether-Polycarbonatdiole können eingesetzt werden.

Die Hydroxylpolycarbonate sollten im wesentlichen linear sein. Sie können jedoch gegebenenfalls durch den Einbau polyfunktioneller Komponenten, insbesondere niedermolekularer Polyole, leicht verzweigt werden. Hierzu eignen sich beispielsweise Glycerin, Trimethylolpropan, Hexantriol-1,2,6, Butantriol-1,2,4, Trimethylolpropan, Pentaerythrit, Chinit, Mannit, Sorbit, Methylglykosid oder 1,3,4,6-Dianhydrohexite. Bevorzugt sind solche Polycarbonate auf Basis von Hexandiol-1,6, sowie modifizierend wirkenden Co-Diolen wie z. B. Butandiol-1,4 oder auch von ε-Caprolacton.

Weitere bevorzugte Polycarbonatdiole sind solche auf Basis von Mischungen aus Hexandiol-1,6 und Butandiol-1,4.

Das Polycarbonatpolyol ist vorzugsweise im Wesentlichen linear ausgebildet und weist nur eine geringfügige dreidimensionale Vernetzung auf.

Geeignete Hydroxylgruppen aufweisenden Polyester sind beispielsweise Umsetzungsprodukte von mehrwertigen, vorzugsweise zweiwertigen Polycarbonsäuren. Anstelle der freien Carbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und / oder heterocyclischer Natur sein und gegebenenfalls, z. B. durch Halogenatome, substituiert und / oder ungesättigt sein. Bevorzugt sind aliphatische und cycloaliphatische Dicarbonsäuren. Beispiele hierfür sind: Bernsteinsäure, Adipinsäure, Azelainsäure, Sebacinsäure, Phthalsäure, Tetrachlorphthalsäure, Isophtalsäure, Terephthalsäure, Tetrahydrophthalsäure, Hexahydrophthalsäure, Cyclohexandicarbonsäure, Itaconsäure, Sebacinsäure, Glutarsäure, Korksäure, 2-Methylbernsteinsäure, 3,3-Diethylglutarsäure, 2,2-Dimethylbernsteinsäure, Maleinsäure, Malonsäure, Fumarsäure oder Terephthalsäuredimethylester. Anhydride dieser Säuren sind ebenfalls, soweit sie existieren, zu verwenden. Beispiele sind Maleinsäureanhydrid, Phthalsäureanhydrid, Tetrahydrophthalsäureanhydrid, Glutarsäureanhydrid, Hexahydrophthalsäureanhydrid und Tetrachlorphthalsäureanhydrid.

Als mehrwertige Alkohole kommen bei der Herstellung der Polyester vorzugsweise Diole zur Anwendung. Beispiele geeigenter Diole sind z. B. Ethylenglykol, Propylenglykol-1,2, Propylenglykol-1,3, Butandiol-1,4, Butandiol-2,3, Diethylenglykol, Triethylenglykol, Hexandiol-1,6, Octandiol-1,8, Neopentylglykol, 2-Methyl-1,3-propandiol oder Hydroxypivalinsäureneopentylglykolester. Auch Polyesterdiole aus Lactonen, z. B. ε-Caprolacton, sind einsetzbar. Als gegebenenfalls mit zu verwendende Polyole sind hier beispielsweise Trimethylolpropan, Glycerin, Erythrit, Pentaerythrit, Trimethylolbenzol oder Trishydroxyethylisocyanurat zu nennen. Die Polyurethanharnstoffe der Haftvermittlerschicht und des Topcoats weisen Einheiten auf, die auf mindestens ein Polyisocyanat als Aufbaukomponente zurückgehen.

Als Polyisocyanate b) und / oder g) können alle dem Fachmann bekannten aromatischen, araliphatischen, aliphatischen und cycloaliphatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder phosgen-freien Verfahren hergestellt sind. Die Polyisocyanate können auch Iminooxadiazindion-, Isocyanurat-, Uretdion-, Urethan-, Allophanat-, Biuret-, Harnstoff-, Oxadiazintrion, Oxazolidinon-, Acylharnstoff und / oder Carbodiimid-Strukturen aufweisen.

Bevorzugt werden jedoch aliphatische und / oder cycloaliphatische Polyisocyanate eingesetzt, wobei diese insbesondere ein Kohlenstoffgrundgerüst (ohne die enthaltenen NCO-Gruppen) von 3 bis 30, bevorzugt 4 bis 20 Kohlenstoffatomen aufweisen können.

Besonders bevorzugte Verbindungen der Komponenten b) und / oder g) sind beispielsweise Bis-(isocyanatoalkyl)ether, Bis- und Tris-(isocyanatoalkyl)benzole, -toluole, sowie -xylole, Propandiisocyanate, Butandiisocyanate, Pentandiisocyanate, Hexandiisocyanate (z.B. Hexamethylendiisocyanat, HDI), Heptandiisocyanate, Octandiisocyanate, Nonandiisocyanate (z.B. Trimethyl-HDI (TMDI) in der Regel als Gemisch der 2,4,4- und 2,2,4-Isomeren), Nonantriisocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodekantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI), Bis-(4-isocyanatocyclohexyl)methan (H₁₂MDI) oder Bis-(isocyanatomethyl)norbornan (NBDI).

Ganz besonders bevorzugt ist, wenn das Polyisocyanat b) und / oder das Polyisocyanat g) eine oder mehrere Verbindungen ausgewählt aus der Gruppe Hexamethylendiisocyanat, Trimethylhexamethylendiisocyanat, 2-Methylpentan-1,5-diisocyanat, Isophorondiisocyanat, 1,3- und / oder 1,4-Bis(isocyanatomethyl)cyclohexan, Bis(isocyanatomethyl)norboman, 3(4)-Isocyanatomethyl-1-methyl-cyclohexylisocyanat, 4,4'-Bis(isocyanatocyclohexyl)methan oder deren Derivate mit Uretdion-, Isocyanurat-, Urethan-, Allophanat-, Biuret-, Iminooxadiazindion- und / oder Oxadiazintrionstruktur mit mehr als zwei NCO-Gruppen umfasst.

Die Polyurethanharnstoffe der Haftvermittlerschicht und des Topcoats weisen weiterhin auch Einheiten auf, die auf mindestens ein Amin mit wenigstens zwei Isocyanat-reaktiven Gruppen zurückgehen.

Als Isocyanat-reaktive Gruppen werden insbesondere Hydroxy-, Amin und / oder Thiol-Gruppen angesehen.

Bevorzugt ist, wenn die Isocyanat-reaktiven Gruppen des Amines c) und / oder des Amines h) Aminogruppen und gegebenenfalls zusätzlich Hydroxygruppen umfassen.

Das Amin c) und / oder das Amin h) kann bevorzugt eine oder mehrere Verbindungen ausgewählt aus der Gruppe Hydrazin, Ethylendiamin, 1,2- Diaminopropan, 1,3-Diaminopropan, 1,4-Diaminobutan, 1,6-Diaminohexan, Isophorondiamin, Isomerengemische von 2,2,4- und 2,4,4-Trimethylhexamethylendiamin, 2-Methylpentamethylendiamin, Diethylentriamin, 1,3-und 1,4-Xylylendiamin, α,α,α',α'-Tetramethyl-1,3- und -1,4-xylylendiamin und 4,4'-Diaminodicyclohexylmethan, Dimethylethylendiamin, Adipinsäuredihydrazid, 1,4-Bis(aminomethyl)cyclohexan, 4,4'-Diamino-3,3'-dimethyldicyclohexylmethan,, N-Aminoethylethanolamin, Ethanolamin, 3-Aminopropanol, Neopentariolamin, Diethanolamin umfassen.

Erfindungsgemäß ist weiterhin vorgesehen, dass der Polyurethanharnstoff der Haftvermittlerschicht und des Topcoats einen monofunktionellen Polyoxyalkylenether d) und / oder i) als Aufbaukomponente umfasst. Die monofunktionellen Polyoxyalkylenether liegen im fertigen Polymer jeweils als Endgruppe vor und bewirken eine Hydrophilierung des Polyurethanharnstoffes.

Geeignete monofunktionelle Polyoxyalkylenether d) und / oder i) sind beispielsweise im statistischen Mittel 5 bis 70, bevorzugt 7 bis 55 Ethylenoxideinheiten pro Molekül aufweisende Polyalkylenoxidpolyetheralkohole, die in an sich bekannter Weise durch Alkoxylierung geeigneter Startermoleküle zugänglich sind (z.B. in Ullmanns Enzyklopädie der technischen Chemie, 4. Auflage, Band 19, Verlag Chemie, Weinheim S. 31-38). Als Startermoleküle können beispielsweise gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol zum Einsatz kommen.

Bevorzugte Startermoleküle sind gesättigte Monoalkohole.

Besonders bevorzugt wird Diethylenglykolmonobutylether als Startermolekül verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

In Weiterbildung der Erfindung ist vorgesehen, dass der monofunktionelle Polyoxyalkylenether d) und / oder der monofunktionelle Polyoxyalkylenether i) mindestens 40 mol-% Ethylenoxid- und maximal 60 mol-% Propylenoxideinheiten umfasst.

Das mittlere Molgewicht der monofunktionellen Polyoxyalkylenether beträgt bevorzugt 500 g/mol bis 5000 g/mol, besonders bevorzugt 1000 g/mol bis 4000 g/mol und ganz besonders bevorzugt 1000 bis 3000 g/mol.

Gemäß einer weiteren Ausführungsform der Erfindung umfasst der Polyurethanharnstoff der Haftvermittlerschicht und / oder des Topcoats zusätzlich Einheiten, die auf mindestens ein niedermolekulares Polyol als Aufbaukomponente zurückgehen. Die zum Aufbau der Polyurethanharnstoffe eingesetzten niedermolekularen Polyole bewirken in der Regel eine Versteifung der Polymerkette. Das Molekulargewicht der niedermolekularen Polyole beträgt bevorzugt 62 bis 500 g/mol, weiter bevorzugt 62 bis 400 g/mol und besonders bevorzugt 62 bis 200 g/mol.

Geeignete niedermolekulare Polyole können aliphatische, alicyclische oder aromatische Gruppen enthalten. Genannt seien hier beispielsweise die niedermolekularen Polyole mit bis zu etwa 20 Kohlenstoffatomen je Molekül, wie z. B. Ethyenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), sowie Trimethylolpropan, Glycerin oder Pentaerythrit und Mischungen dieser und gegebenenfalls auch weiterer niedermolekularer Polyole. Auch Esterdiöle wie z. B. α-Hydroxybutyl-ε-hydroxy-capronsäureester, ω-Hydroxyhexyl-γ-hydroxybuttersäure-ester, Adipinsäure-(ß-hydroxyethyl)ester oder Terephthalsäure-bis(ß-hydroxyethyl)-ester können verwendet werden.

Gemäß einer weiteren bevorzugten Ausführungsform der Erfindung können die Isocyanatreaktiven Gruppen der Carbonsäure e) Hydroxy- und / oder Aminogruppen sein.

Vorteilhaft ist auch, wenn die Carbonsäure e) eine oder mehrere Verbindungen ausgewählt aus der Gruppe der Monohydroxycarbonsäuren, Dihydroxycarbonsäuren, Trihydroxycarbonsäuren, Monoaminocarbonsäuren, Diaminocarbonsäuren, Triaminocarbonsäuren umfasst.

Ganz besonders bevorzugt kann die Carbonsäure e) eine oder mehrere Verbindungen ausgewählt aus der Gruppe Dimethylolpropionsäure, Dimethylolbuttersäure, Dimethylolessigsäure, Dihydroxybemsteinsäure, Hyxdroxypivalinsäure, Hydroxyessigsäure, Hydroxypropionsäure, 6-Aminohexansäure, Alanin, N-(2-Amminoethyl)-ß-alanin, Aminoundecansäure, 8-Aminooctansäure, 5-Aminopentansäure, 4-Amino-buttersäure, Aminobenzoesäure, 4-Aminomethylcyclohexancarbonsäure, 2-Aminohexansäure, 4-Aminocyclohexan-carbonsäure, 12-Aminododecansäure, 9-Aminononacarbonsäure umfassen.

Die durch die Carbonsäure e) in den Polyurethanharnstoff eingebrachte Carbonsäuregruppe kann auch noch in einem weitern Schritt in eine Carboxylat-Gruppe umgewandelt werden. Dazu kann der Polyurethanharnstoff mit einem Neutralisationsmittel zur Reaktion gebracht werden, wobei bevorzugt tertiäre Amine wie Triethylamin, Tripropylamin, Tributylamin, Triisopropanolamin, Triethanolamin, N,N-Dimethyethanolamin oder Ammoniak oder Alkalihydroxide wie Natrium- oder Kaliumhydroxid oder Alkalicarbonate oder - hydrogencarbonate zum Einsatz kommen können.

Die erfindungsgemäßen Polyurethanharnstoffe der Haftvermittlerschicht und / oder des Topcoats können darüber hinaus weitere, für den angestrebten Zweck übliche Bestandteile wie Additive und Füllstoffe, enthalten. Ein Beispiel hierfür sind pharmakologische Wirkstoffe und Additive, die die Freisetzung von pharmakologischen Wirkstoffen fördern ("drug-eluting-Additive").

Pharmakologische Wirkstoffe, die insbesondere in den erfindungsgemäßen Polyurethanharnstoff des Topcoats verwendet werden können, sind beispielsweise thromboresistente Mittel, antibiotische Mittel, Antitumormittel, Wachtumshormone, Antivirusmittel, antiangiogene Mittel, angiogene Mittel, antimitotische Mittel, entzündungshemmende Mittel, Zellzyklusregulierende Mittel, genetische Mittel, Hormone, sowie deren Homologe, Derivate, Fragmente, pharmazeutische Salze und Kombinationen.

Spezifische Beispiele derartiger pharmakologischer Wirkstoffe bzw. Arzneimittel schließen somit thromboresistente (nichtthrombogene) Mittel bzw. andere Mittel zur Unterdrückung einer akuten Thrombose, Stenose oder späten Re-Stenose der Arterien ein. Dies sind zum Beispiel Heparin, Streptokinase, Urokinase, Gewebe-Plasminogen-Aktivator, Anti-Thromboxan-B₂-Mittel; Anti-B-Thromoboglobulin, Prostaglandin-E, Aspirin, Dipyridimol, Anti-Thromboxan-A₂-Mittel, muriner monoklonaler Antikörper 7E3, Triazolopyrimidin, Ciprosten, Hirudin, Ticlopidin, Nicorandil usw. Ein Wachstumsfaktor kann ebenfalls als ein Arzneimittel benutzt werden, um unterintimale fibromuskülare Hyperplasie an der arteriellen Stenosestelle zu unterdrücken, bzw. es kann jeder beliebige andere Hemmstoff des Zellwachstums an der Stenosestelle zum Einsatz kommen.

Der pharmakologische Wirkstoff kann auch aus einem Vasodilatator bestehen, um Vasospasmus entgegen zu wirken. Ein Beispiel für ein solches Antispasmusmittel ist Papaverin.

Das Wirkstoff kann auch ein vasoaktives Mittel an sich wie Calciumantagonisten oder α- und β-adrenergische Agonisten oder Antagonisten sein. Zusätzlich kann der Wirkstoff ein biologisches Haftmittel wie Cyanoacrylat in medizinischer Qualität oder Fibrin, das beispielsweise für das Ankleben einer Gewebeklappe an die Wand einer Koronararterie verwendet wird, umfassen.

Der pharmakologische Wirkstoff kann ferner ein antineoplastisches Mittel wie 5-Fluorouracil, vorzugsweise mit einem kontrollierenden freisetzenden Träger für das Mittel, sein. Dies eignet sich insbesondere, um ein antineoplastisches Mittel lokal, kontinuierlich und kontrolliert im Bereich eines Tumors freizusetzen.

Der pharmakologischer Wirkstoff kann auch ein Antibiotikum, vorzugsweise in Kombination mit einem kontrollierenden freisetzenden Träger für die fortdauernde Freisetzung aus der Beschichtung eines medizinischen Geräts an einen lokalisierten Infektionsherd innerhalb des Körpers, sein. In ähnlicher Weise kann das therapeutische Mittel Steroide für den Zweck des Unterdrückens einer Entzündung in lokalisiertem Gewebe enthalten.

Spezifische Beispiele geeigneter pharmakologischer Wirkstoffe umfassen:
(a) Heparin, Heparinsulfat, Hirudin, Hyaluronsäure, Chondroitinsulfat, Dermatansulfat, Keratansulfat, lytische Mittel, einschließlich Urokinase und Streptokinase, deren Homologe, Analoge, Fragmente, Derivate und pharmazeutische Salze;
(b) antibiotische Mitteln wie Penicilline, Cephalosprine, Vacomycine, Aminoglycoside, Quinolone, Polymxine, Erythromycine; Tertracycline, Chloramphenicole, Clindamycine, Lincomycine, Sulfonamide, deren Homologe, Analoge, Derivate, pharmazeutische Salze und Mischungen;
(c) Paclitaxel, Docetaxel, Immunsuppressiva wie Sirolimus oder dem Sirolimus verwandte Limus-Derivate wie beispielsweise Everolimus, Biolimus A9, Tacrolimus oder Zotarolimus, Alkylierungsmittel einschließlich Mechlorethamin, Chlorambucil, Cyclophosphamid, Melphalan und Ifosfamid; Antimetaboliten einschließlich Methotrexat, 6-Mercaptopurin, 5-Fluorouracil und Cytarabin; Pflanzenalkoide einschließlich Vinblastin; Vincristin und Etoposid; Antibiotika einschließlich Doxorubicin, Daunomycin, Bleomycin und Mitomycin; Nitrosurea einschließlich Carmustin und Lomustin; anorganische Ionen einschließlich Cisplatin; biologische Reaktionsmodifikatoren einschließlich Interferon; angiostatinische Mittel und endostatinische Mittel; Enzyme einschließlich Asparaginase; und Hormone einschließlich Tamoxifen und Flutamid, deren Homologe, Analoge, Fragmente, Derivate, pharmazeutische Salze und Mischungen; und
(d) antivrale Mittel wie Amantadin, Rimantadin, Rabavirin, Idoxuridin, Vidarabin, Trifluridin, Acyclovir, Ganciclocir, Zidovudin, Phosphonoformate, Interferone, deren Homologe, Analoge, Fragmente, Derivate, pharmazeutische Salze und Mischungen; und
(e) entzündungshemmende Mittel wie beispielsweise Ibuprofen, Dexamethason, Methylprednisolon deren Homologe, Analoge, Derivate, pharmazeutische Salze und Mischungen.

Weitere übliche Zusatzstoffe und Hilfsmittel wie Verdickungsmittel, Griffhilfsmittel, Pigmente, Farbstoffe, Mattierungsmittel, UV-Stabilisatoren, phenolische Antioxidantien, Lichstabilisatoren, Hydrophobierungsmittel und / oder Verlaufshilfsmittel können ebenfalls in den erfindungsgemäßen Polyurethanharnstoffen mitverwendet werden.

Gemäß einer weiteren bevorzugten Ausführungsform der Erfindung bedeckt der Topcoat die Haftvermittlerschicht vollständig. Alternativ kann der Topcoat aber auch nur bereichsweise auf die Haftvermittlerschicht aufgebracht sein.

Bei einer weiteren bevorzugten Ausführungsform des erfindungsgemäßen Schichtaufbaus ist die Haftvermittlerschicht auf ein Substrat und bevorzugt auf ein medizinisches Gerät aufgebracht. Besonders bevorzugt ist, wenn die Haftvermittlerschicht auf der gesamten Oberfläche des Substrates aufgebracht ist

Das Substrat kann beispielsweise Metalle, Textilien, Glas, Keramiken und / oder Kunststoffe enthalten oder daraus bestehen. Besonders bevorzugt besteht das Substrat jedoch aus einem Metall oder aus einem Kunststoff.

Beispiele für Metalle sind Edelstahl oder Nickel-Titan-Legierungen.

Bei den Kunststoffen kann es sich um Polyamide, Blockcopolymere aus Styrol und ungesättigten Verbindungen wie Ethylen, Butylen und Isopren, Polyethylene oder Copolymere aus Polyethylen und Polypropylen, Silikone, Polyvinylchloride (PVC) und / oder Polyurethane handeln.

Die Bezeichnung "medizinisches Gerät" ist im Rahmen der vorliegenden Erfindung breit zu verstehen. Geeignete, nicht einschränkende Beispiele für medizinische Geräte (einschließlich Instrumente) sind Kontaktlinsen; Kanülen; Katheter, zum Beispiel urologische Katheter wie Blasenkathetern oder Harnleiterkatheter; zentralvenöse Katheter; venöse Katheter oder Einlass- bzw. Auslass-Katheter; Dilatationsballons; Katheter für die Angioplastie und die Biopsie; Katheter, die für das Einbringen eines Stents, eines Propf- oder eines Kavafilters verwendet werden; Ballonkatheter oder andere dehnbare medizinische Geräte; Endoskope; Lamygoskope; Trachealgeräte wie Endotrachealschläuche, Atemgeräte und andere Trachealabsauggeräte; bronchoalveolare Spülungskatheter; Katheter, die bei der Koronarangioplastie verwendet werden; Führungsdrähte, Einführer und Ähnliches; Gefäßpfropfen; Schrittmacherteile; Cochleaimplantate; Zahnimplantatschläuche für die Nahrungszufuhr, Dränageschläuche; und Führungsdrähte.

Besonders bevorzugt ist der erfindungsgemäße Schichtaufbau jedoch auf einen Katheter, einen Führungsdraht, ein Endoskop, einen Stent, einen Dilatationsballon oder auf einen Blutfilter aufgebracht.

Die Haftvermittlerschicht kann insbesondere eine Dicke im Bereich von 1 bis 200 µm, bevorzugt von 1 bis 150 µm und besonders bevorzugt von 1 bis 100 µm aufweisen.

Die hydrophile Gleitschicht kann wiederum insbesondere eine Dicke im Bereich von 1 bis 500 µm, bevorzugt von 1 bis 250 µm und besonders bevorzugt von 5 bis 100 µm aufweisen.

Der Schichtverbund kann noch weitere Schichten umfassen. Dabei kann es sich beispielsweise um eine oder mehrere wirkstoffhaltige Schichten handeln, die zumindest bereichsweise mit dem Topcoat und / oder der Haftvermittlerschicht verbunden sein können.

Ein weiterer Gegenstand der Erfindung ist ein Verfahren zur Herstellung eines erfindungsgemäßen Schichtverbundes bei dem die Haftvermittlerschicht auf ein Substrat und auf die Haft-vermittlerschicht zumindest bereichsweise die hydrophile Gleitschicht aufgebracht wird.

Die Polyurethanharnstoffe der Haftvermittlerschicht und / oder des Topcoats können beispielweise auf folgendem Weg hergestellt werden: Zunächst werden in einem ersten Schritt die Komponenten a), b) d) und e) bzw. f), g), und i) jeweils zu einem Prepolymer umgesetzt, dass freie Isocyanatgruppen aufweist und gleichzeitig frei von Harnstoffgruppen ist. Dabei werden die Stoffmengen der Komponenten bevorzugt so gewählt, dass das Verhältnis von Isocyanatgruppen zu Isocyanat-reaktiven-Gruppen 0,8 bis 3,5, bevorzugt 0,9 bis 3,0 besonders bevorzugt 1,0 bis 2,5 beträgt. Anschließend wird das Prepolymer mit dem Amin c) bzw. h) umgesetzt, wobei das Äquivalentverhältnis der isocyanatreaktiven Gruppen der Amine zu den freien Isocyanat-Gruppen des Prepolymers zwischen bevorzugt 40 bis 150 %, weiter bevorzugt zwischen 50 bis 120 % und besonders bevorzugt zwischen 60 bis 120 %, betragen kann.

Die erfindungsgemäßen Polyurethanharnstoffe können auch nach dem sogenannte Aceton-Verfahren als Dispersion hergestellt.

Die Carbonsäuregruppen im Polyurethanharnstoff der Hafrvermittlerschicht können optional durch Umsetzung mit basischen Verbindungen zu Carboxylatanionen umgesetzt werden. Diese Umsetzungsschritt kann zu verschiedenen Zeiten der Polymeraufbaureaktion durchgeführt werden, wobei er bei dem Aceton-Verfahren bevorzugt vor der Kettenverlängerungsreaktion mit dem Amin c) durchgeführt wird.

Die Haftvermittlerschicht und / oder der Topcoat können durch verschiedenen Techniken auf Substrate aufgetragen werden. Dazu kann beispielsweise eine Lösung oder eine Dispersion des jeweiligen Polyurethanharnstoff durch Sprühen, Tauchen, Rakeln, Drucken oder Transferbeschichtung aufgebracht werden. Anschließend wird die Beschichtung getrocknet.

Ebenfalls Gegenstand der Erfindung ist ein nach dem Verfahren hergestellter Schichtverbund.

Die Erfindung wird im Folgenden anhand von Beispielen näher erläutert.

### Beispiele:

### Methoden:

Die Ermittlung des NCO-Gehaltes der in den Beispielen und Vergleichsbeispielen beschriebenen Verbindungen erfolgte durch Titration gemäß DIN EN ISO 11909.

Die Bestimmung der Festkörpergehalte erfolgte nach DIN-EN ISO 3251. Es wurden 1 g Polyurethandispersion bei 115 °C bis zur Gewichtskonstanz (15-20 min) mittels eines Infrarottrockners getrocknet.

Die Messung der mittleren Teilchengrößen der Polyurethandispersionen erfolgt mit Hilfe des High Performance Particle Sizer (HPPS 3.3) der Firma Malvern Instruments.

Die in % angegebenen Mengenangaben verstehen sich, wenn nicht anders vermerkt, als Gew.-% und beziehen sich auf die erhaltene wässrige Dispersion.

### Verwendete Substanzen und Abkürzungen:

| | |
|---|---|
| Desmophen C2200: | Polycarbonatpolyol, OH-Zahl 56 mg KOH/g, zahlenmittleres Molekulargewicht 2000 g/mol (Bayer, MaterialScience AG, Leverkusen, DE) |
| Polyether LB 25: | monofunktioneller Polyether auf Ethylenoxid-/Propylenoxidbasis (zahlenmittleres Molekulargewicht 2250 g/mol, OH-Zahl 25 mg KOH/g (Bayer MaterialScience AG, Leverkusen, DE) |

### Beispiel 1: Herstellung einer Polyurethanharnstoff-Dispersion für eine Haftvermittlerschicht

277,2 g Desmophen C 2200, 33,1 g Polyether LB 25 und 8,7 g Dimethylolpropionsäure wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 16 h war der theoretische NCO-Gehalt erreicht. Das fertige Prepolymer wurde bei 50 °C in 711 g Aceton gelöst. Bei 40 °C wurden 6,4 g Triethylamin innerhalb von einer Minute zugetropft und dann 10 min nachgerührt. Anschließend wird ebenfalls bei 40 °C eine Lösung aus 4,8 g Ethylendiamin in 16 g Wasser innerhalb von 10 min zudosiert. Die Nachrührzeit betrug 15 min. Anschließend wurde innerhalb von 15 min durch Zugabe von 1253 g Wasser dispergiert. Es folgte die Entfernung des Lösungsmittels durch Destillation im Vakuum. Es wurde eine lagerstabile Polyurethandispersion mit einem Festkörpergehalt von 24,4 % und einer mittleren Teilchengröße von 78 nm erhalten.

### Beispiel 2: Polyurethanharnstoff-Dispersion für eine hydrophilie Beschichtung

277,2 g Desmophen C 2200, 33,1 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 3 h 40 min war der theoretische NCO Wert erreicht. Das fertige Prepolymer wurde bei 50 °C in 711 g Aceton gelöst und anschließend bei 40 °C eine Lösung aus 4,8 g Ethylendiamin in 16 g Wasser innerhalb von 10 min zudosiert. Die Nachrührzeit betrug 15 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 41,5 % und einer mittleren Teilchengröße von 164 nm erhalten.

### Beispiel 3: Polyurethanharnstoff-Dispersion für eine hydrophile Beschichtung

135,0 g Desmophen C2200, 33,8 g eines Polycarbonatdiols, hergestellt aus 3(4),8(9)-Bis(hydroxymethyl)-tricyclo(5.2.1.0/2.6)decan (TCD-Alkohol) und Dimethylcarbonat mit einem zahlenmittleren Molgewicht von 500 g/mol, 49,7 g Polyether LB 25 und 6,7 g Neopentylglykol wurden bei 65 °C vorgelegt und 5 min durch Rühren homogenisiert. Zu dieser Mischung gab man bei 65 °C innerhalb von 1 min zuerst 71,3 g 4,4'-Bis(isocyanatocyclohexyl)methan (H₁₂MDI) und danach 11,9 g Isophorondiisocyanat. Man erwärmte auf 110 °C. Nach 20 h war der theoretische NCO-Wert erreicht. Das fertige Prepolymer wurde bei 50 °C in 590 g Aceton gelöst und anschließend bei 40 °C eine Lösung aus 4,8 g Ethylendiamin in 16 g Wasser innerhalb von 10 min zudosiert. Die Nachrührzeit betrug 5 min. Anschließend wurde innerhalb von 15 min durch Zugabe von 590 g Wasser dispergiert. Es folgte die Entfernung des Lösungsmittels durch Destillation im Vakuum. Es wurde eine lagerstabile Polyurethandispersion mit einem Festkörpergehalt von 33,7 % und einer mittleren Teilchengröße von 83 nm erhalten.

### Beispiel 4: Polyurethanharnstoff-Dispersion für eine hydrophile Beschichtung

195,4 g Desmophen C 2200, 30,0 g LB 25 und 47,8 g 4,4'-Bis(isocyanatocyclohexyl)methan (H₁₂MDI) wurden bei 110 °C bis zu einem konstanten NCO-Gehalt von 2,3 % umgesetzt. Man ließ abkühlen und verdünnte mit 350,0 g Toluol und 200 g iso-Propanol. Bei Raumtemperatur wurde eine Lösung von 12,7 g Isophorondiamin in 94,0 g 1-Methoxypropanol-2 zugegeben. Nach beendetem Aufbau des Molgewichtes und Erreichen des gewünschten Viskositätsbereiches rührte man weitere 4 Stunden, um den restlichen Isocyanatgehalt mit iso-Propanol zu blockieren. Man erhielt 930 g einer 30,7%igen Polyurethanharnstofflösung in Toluol/iso-Propanol/1-Methoxypropanol-2 mit einer Viskosität von 38600 mPas bei 22 °C.

### Beispiel 5: Polyurethanharnstoff-Dispersion für eine hydrophile Beschichtung

97,8 g Desmophen C 2200, 24,5 g eines Polycarbonatdiols, hergestellt aus 3(4),8(9)-Bis(hydroxymethyl)-tricyclo(5.2.1.0/2.6)decan (TCD-Alkohol) und Dimethylcarbonat mit einem zahlenmittleren Molgewicht von 500 g/mol, 40,0 g LB 25 und 47,8 g 4,4'-Bis(isocyanatocyclohexyl)methan (H₁₂MDI) wurden bei 110 °C bis zu einem konstanten NCO-Gehalt von 3,0 % umgesetzt. Man ließ abkühlen und verdünnte mit 300,0 g Toluol und 180 g iso-Propanol. Bei Raumtemperatur wurde eine Lösung von 12,5 g Isophorondiamin in 100 g 1-Methoxypropanol-2 zugegeben. Nach beendetem Aufbau des Molgewichtes und Erreichen des gewünschten Viskositätsbereiches rührte man weitere 4,5 Stunden, um den restlichen Isocyanatgehalt mit iso-Propanol zu blockieren. Man erhielt 803 g einer 28,4%igen Polyurethanharnstofflösung in Toluol/iso-Propanol/1-Methoxypropanol-2 mit einer Viskosität von 3250 mPas bei 22 °C.

### Beispiel 6: Metallplättchen mit Haftvermittlerschicht

Als Substrat für die Beschichtungen wurde ein Metallplättchen (medizinischer Edelstahl 316 L) der Größe 25x75 mm verwendet. Dieses wurden zunächst mit Hilfe von Isopropanol gereinigt. Hierzu wurden die Metallplättchen 1 h bei Raumtemperatur in Isopropanol gelagert und dann 1 h bei 50 °C getrocknet. Anschließend wurde das Metallplättchen auf dem Probenteller eines Spincoaters (RC5 Gyrset 5, Karl Süss, Garching, Deutschland) eingespannt und mit ca. 2,5 - 3 g der wässrigen unverdünnten Polyurethandispersion des Beispiels 1 homogen bedeckt. Durch Rotation des Probentellers für 20 sec bei 1300 Umdrehungen pro Minute erhielt man eine homogene Beschichtung, die 15 min bei 100 °C und danach 24 h bei Raumtemperatur getrocknet wurde.

### Beispiel 7: Metallplättchen mit hydrophiler Beschichtung

Ein weiteres Metallplättchen wurde analog zu Beispiel 6 mit der hydrophilen Polyurethanharnstoffdispersion des Beispiels 2 beschichtet

### Beispiel 8: Nasshaftungsprüfung

Die erhaltenen beschichteten Metallplättchen der Beispiele 6 und 7 wurden nach vollständigem Trocknen für 24 h in vollentsalztem Wasser bedeckend gelagert, um nasse Beschichtungen zu erhalten. Anschließend wurden die Metallplättchen aus dem Wasser entnommen. Durch vorsichtiges Reiben mit einem Finger wurde dann die Nasshaftung der jeweiligen Beschichtungen geprüft. Die hydrophile Beschichtung des Beispiels 7 konnte leicht vom Metallplättchen gelöst werden. Sie wies somit keine ausreichende Nasshaftung auf. Die Beschichtung des Beispiels 3 konnte hingegen nicht von der metallischen Unterlage entfernt werden. Sie wies folglich eine sehr gute Nasshaftung auf.

### Beispiel 9: Herstellung eines erfindungsgemäßen Schichtverbundes

Das bereits mit der Haftvermittlerschicht versehene Metallplättchen des Beispiels 6 wurde zusätzlich mit einer hydrophilen Beschichtung versehen. Dazu wurde die Polyurethanharnstoff-Dispersion des Beispiels 2 analog zu Bespiel 7 auf die Haftvermittlerschicht aufgebracht und getrocknet. Auf diese Weise wurde ein Schichtverbund bestehend aus einem metallischen Substrat, einer Haftvermittlerschicht und einem hydrophilen Topcoat erhalten.

### Beispiel 10: Nasshaftungsprüfung

Analog zum Beispiel 8 wurde die Nasshaftung des Schichtverbunds des Beispiels 9 geprüft. Dabei wurde festegestellt, dass sich der Schichtverbund nicht durch Reiben mit dem Finger von der Metalloberfläche ablösen ließ. Somit konnte gezeigt werden, dass durch die Haftvermittlerschicht die Haftung der hydrophilen Beschichtung auf Metall deutlich verbessert werden konnte.

### Beispiel 11: Kontaktwinkelmessungen

An den Beschichtungen der Beispiele 7 und 9 wurden statische Kontaktwinkelmessungen mit Wasser durchgeführt. Der Kontaktwinkel von Wasser auf den Substraten wurde bei 23°C mit dem Data Physics Contact Angle System OCA bestimmt. Es wurde destilliertes Wasser benutzt, das mit einer Spritze mit einer Kanüle mit einem Innendurchmesser von 100 µm mit einer Rate von 18 µl/s zu 3 µl Tröpfchen appliziert wurde. Die Auswertung des Kontaktwinkels erfolgte mittels der Data Physics Software. Es wurden für jede Probe 5 Messungen durchgeführt, woraus ein Mittelwert des Kontaktwinkels gebildet wurde. In dem Fall, dass einer der Messwerte um mehr als 0,5° vom Mittelwert abwich, wurde dieser Messwert durch eine weitere Messung ersetzt.

Für die Beschichtung des Beispiels 7 wurde ein Wasserrandwinkel von 49 ° gemessen. Der Wasserrandwinkel des Schichtverbundes des Beispiels 9 betrug 25 °.

Die Ergebnisse zeigen, dass die Beschichtung des Beispiels 7 allein einen relativ hohen Wasserrandwinkel aufweist. Durch Überlackieren mit einer hydrophilen Beschichtung erhält man dann aber einer Schichtverbund mit einer stark hydrophilien Oberfläche, der auch eine starke Nasshaftfestigkeit aufweist, wie das Beispiel 10 zeigt.

### Beispiel 12: Schichtverbünde mit unterschiedlichen Topcoats

Metallplättchen, die analog zu Beispiel 6 mit einer Haftvermittlerschicht versehen worden waren, sind anschließend jeweils analog zu Beispiel 9 mit einer hydrophilen Beschichtung versehen worden. Dazu wurde jeweils eines der Materialien der Beispiele 3,4 und 5 mit einem 200 µm Rakel aufgerakelt, 15 min bei 100 °C und 24 h bei Raumtemperatur getrocknet.

### Beispiel 13: Nasshaftungsprüfung

Analog zum Beispiel 10 wurde die Nasshaftung der Schichtverbunde des Beispiels 12 geprüft, wobei Beschichtungen abweichend für drei Tage bei Raumtemperatur in vollentsalztes Wasser eingetaucht gelagert wurden. Die Beschichtungen konnten durch Reiben nicht vom Metalluntergrund abgelöst werden.

### Beispiel 14: Vergleich zu bekannten Haftvermittlerschichten

O. Negele und W. Funke berichten in Progress in Organic Coatings 28 (1996), 285 - 289, dass Polycarbonsäuren, speziell Polyacrylsäuren, ganz allgemein die Nassfestigkeit von Beschichtungen stark verbessern können. Aus diesem Grund wurde zum Vergleich die Haftung einer Beschichtung aus der hydrophilien Verbindung des Beispiels 2 auf Polyacrylsäuren untersucht. Dazu wurden zwei kommerziell erhältliche Poylacrylsäuren (Polyacrylsäure 35 Gew% in Wasser, gewichtsmittleres Molgewicht M_{w} ca. 250000, Aldrich Artikel-Nr. 416002 sowie Polyacrylsäure 25 Gew% in Wasser, teilweise Na-Salz, gewichtsmittleres Molgewicht M_{w} ca. 240000, Aldrich Artikel-Nr. 192058) in 50%iger Verdünnung der Ausgangskonzentration mittels Spincoating wie im Beispiel 6 beschrieben auf Metallplättchen beschichtet.

Die Nasshaftung der so hergestellten Beschichtungen wurde analog zu Beispiel 8 geprüft, wobei festgestellt wurde, dass die Beschichtungen nicht vom Metall abgelöst werden konnten und somit eine gute Haftung zeigten.

Anschließend wurden die Metallplättchen mit den bereits vorhandenen Polyacrylsäurebeschichtungen nach dem Trocknen mit einer hydrophilen Beschichtung des Beispiels 2 überlackiert. Wie in Beispiel 12 wurde dazu die hydrophile Beschichtung mit einem 200 µm Rakel aufgetragen.

Die auf die beiden Pblyacrylsäurebeschichtungen aufgetragenen Schichten der hydrophilen Verbindung des Beispiels 2 waren rau und inhomogen. Nach 24 h Wasserlagerung wiesen diese Beschichtungen dann auch keine Haftung zum metallischen Substrat mehr auf.

Dieses Vergleichsbeispiel zeigt, dass nicht jede auf Metall gut haftende Polycarbonsäurebeschichtung als Haftvermittlerschicht für hydrophile Topcoats zu verwenden ist.

## Patentansprüche

1. Schichtverbund umfassend eine Haftvermittlerschicht und einen damit verbundenen hydrophilen Topcoat, wobei die Haftvermittlerschicht einen Polyurethanharnstoff umfasst, der durch Umsetzung wenigstens folgender Komponenten erhältlich ist:
a) einem Makropolyol;
b) einem Polyisocyanat;
c) einem Amin mit wenigstens zwei Isocyanat-reaktiven Gruppen;
d) einem monofunktionellen Polyoxyalkylenether; und
e) einer Carbonsäure mit wenigstens zwei Isocyanat-reaktiven Gruppen,
der hydrophile Topcoat einen Polyurethanharnstoff umfasst, der durch Umsetzung wenigstens folgender Komponenten erhältlich ist:
f) einem Makropolyol;
g) einem Polyisocyanat;
h) einem Amin mit wenigstens zwei Isocyanat-reaktiven Gruppen; und
i) einem monofunktionellen Polyoxyalkylenether;
und der Polyurethanharnstoff des hydrophilen Topcoats keine Carbonsäuregruppen aufweist.

2. Schichtverbund nach Anspruch 1, **dadurch gekennzeichnet, dass** das Makropolyol a) und / oder das Makropolyol f) eine oder mehrere Verbindungen ausgewählt aus der Gruppe Polyesterpolyol, Polyetherpolyol, Polycarbonatpolyol umfasst.

3. Schichtverbund nach einem der Ansprüche 1 oder 2, **dadurch gekennzeichnet, dass** das Polyisocyanat b) und / oder das Polyisocyanat g) eine oder mehrere Verbindungen ausgewählt aus der Gruppe Hexamethylendiisocyanat, Trimethylhexamethylendiisocyanat, 2-Methylpentan-1,5-diisocyanat, Isophorondiisocyanat, 1,3- und / oder 1,4-Bis(isocyanatomethyl)cyclohexan, Bis(isocyanatomethyl)norbornan, 3(4)-Isocyanatomethyl-1-methyl-cyclohexylisocyanat, 4,4'-Bis(isocyanatocyclohexyl)-methan oder deren Derivate mit Uretdion-, Isocyanurat-, Urethan-, Allophanat-, Biuret-, Iminooxadiazindion- und / oder Oxadiazintrionstruktur mit mehr als zwei NCO-Gruppen umfasst.

4. Schichtverbund nach einem der Ansprüche 1 bis 3, **dadurch gekennzeichnet, dass** die Isocyanat-reaktiven Gruppen des Amines c) und / oder des Amines h) Aminogruppen und gegebenenfalls zusätzlich Hydroxygruppen umfassen.

5. Schichtverbund nach einem der Ansprüche 1 bis 4, **dadurch gekennzeichnet, dass** das Amin c) und / oder das Amin h) eine oder mehrere Verbindungen ausgewählt aus der Gruppe Hydrazin, Ethylendiamin, 1,2- Diaminopropan, 1,3-Diaminopropan, 1,4-Diaminobutan, 1,6-Diaminohexan, Isophorondiamin, Isomerengemische von 2,2,4-und 2,4,4-Trimethylhexamethylendiamin, 2-Methylpentamethylendiamin, Diethylentriamin, 1,3- und 1,4-Xylylendiamin, α,α,α',α',-Tetramethyl-1,3- und -1,4-xylylendiamin und 4,4'-Diaminodicyclohexylmethan, Dimethylethylendiamin, Adipinsäuredihydrazid, 1,4-Bis(aminomethyl)cyclohexan, 4,4'-Diamino-3,3'-dimethyldicyclohexylmethan, N-Aminoethylethanolamin, Ethanolamin, 3-Aminopropanol, Neopentanolamin, Diethanolamin umfasst.

6. Schichtverbund nach einem der Ansprüche 1 bis 5, **dadurch gekennzeichnet, dass** der monofunktionelle Polyoxyalkylenether d) und / oder der monofunktionelle Polyoxyalkylenether i) Ethylenoxid- und Propylenoxideinheiten umfasst.

7. Schichtverbund nach Anspruch 6, **dadurch gekennzeichnet, dass** der monofunktionelle Polyoxyalkylenether d) und / oder der monofunktionelle Polyoxyalkylenether i) mindestens 40 mol-% Ethylenoxid- und maximal 60 mol-% Propylenoxideinheiten umfasst.

8. Schichtverbund nach einem der Ansprüche 1 bis 7, **dadurch gekennzeichnet, dass** das mittlere Molgewicht des monofunktionellen Polyoxyalkylenether d) und / oder des monofunktionellen Polyoxyalkylenether i) 500 g/mol bis 5000 g/mol, bevorzugt 1000 g/mol bis 4000 g/mol und besonders bevorzugt 1000 bis 3000 g/mol beträgt.

9. Schichtverbund nach einem der Ansprüche 1 bis 8, **dadurch gekennzeichnet, dass** die Isocyanat-reaktiven Gruppen der Carbonsäure e) Hydroxy- und / oder Aminogruppen sind.

10. Schichtverbund nach einem der Ansprüche 1 bis 9, **dadurch gekennzeichnet, dass** die Carbonsäure e) eine oder mehrere Verbindungen ausgewählt aus der Gruppe der Monohydroxycarbonsäuren, Dihydroxycarbonsäuren, Trihydroxycarbonsäuren, Monoaminocarbonsäuren, Diaminocarbonsäuren, Triaminocarbonsäuren umfasst.

11. Schichtverbund nach einem der Ansprüche 1 bis 9, **dadurch gekennzeichnet, dass** die Carbonsäure e) eine oder mehrere Verbindungen ausgewählt aus der Gruppe Dimethylolpropionsäure, Dimethylolbuttersäure, Dimethylolessigsäure, Dihydroxybernsteinsäure, Hyxdroxypivalinsäure, Hydroxyessigsäure, Hydroxypropionsäure, 6-Aminohexansäure, Alanin, N-(2-Amminoethyl)-ß-alanin, Aminoundecansäure, 8-Aminooctansäure, 5-Aminopentansäure, 4-Amino-buttersäure, Aminobenzoesäure, 4-Aminomethylcyclohexancarbonsäure, 2-Aminohexansäure, 4-Aminocyclohexancarbonsäure, 12-Aminododacansäure, 9-Aminoononacarbonsäure umfasst.

12. Schichtverband nach einem der Ansprüche 1 bis 11, **dadurch gekennzeichnet, dass** die Haftvermittlerschicht auf ein Substrat, bevorzugt auf ein medizinisches Gerät und besonders bevorzugt auf einen Katheter, einen Führungsdraht, ein Endoskop, einen Stent, einen Dilatationsballon oder auf einen Blutfilter aufgebracht ist.

13. Schichtverband nach einem der Ansprüche 1 bis 12, **dadurch gekennzeichnet, dass** die Haftvermittlerschicht eine Dicke im Bereich von 1 bis 200 µm, bevorzugt von 1 bis 150 µm und besonders bevorzugt von 1 bis 100 µm aufweist.

14. Schichtverband nach einem der Ansprüche 1 bis 13, **dadurch gekennzeichnet, dass** die hydrophile Gleitschicht eine Dicke im Bereich von 1 bis 500 µm, bevorzugt von 1 bis 250 µm und besonders bevorzugt von 5 bis 100 µm aufweist.

15. Verfahren zur Herstellung eines Schichtverbundes nach einem der Ansprüche 1 bis 14, **dadurch gekennzeichnet, dass** die Haftvermittlerschicht auf ein Substrat und auf die Haftvermittlerschicht zumindest bereichsweise die hydrophile Gleitschicht aufgebracht wird.

## Claims

1. Layer composite comprising an adhesion promoter layer and a hydrophilic topcoat joined thereto, the adhesion promoter layer comprising a polyurethane urea which is obtainable by reaction of at least the following components:
a) a macropolyol;
b) a polyisocyanate;
c) an amine having at least two isocyanate-reactive groups;
d) a monofunctional polyoxyalkylene ether; and
e) a carboxylic acid having at least two isocyanate-reactive groups;
the hydrophilic topcoat comprising a polyurethane urea which is obtainable by reaction of at least the following components:
f) a macropolyol;
g) a polyisocyanate;
h) an amine having at least two isocyanate-reactive groups; and
i) a monofunctional polyoxyalkylene ether;
and the polyurethane urea of the hydrophilic topcoat having no carboxylic acid groups.

2. Layer composite according to Claim 1, **characterized in that** the macropolyol a) and/or the macropolyol f) comprise/comprises one or more compounds selected from the group of polyester polyol, polyether polyol, and polycarbonate polyol.

3. Layer composite according to either of Claims 1 and 2, **characterized in that** the polyisocyanate b) and/or the polyisocyanate g) comprise/comprises one or more compounds selected from the group of hexamethylene diisocyanate, trimethylhexamethylene diisocyanate, 2-methylpentane 1,5-diisocyanate, isophorone diisocyanate, 1,3- and/or 1,4-bis(isocyanatomethyl)cyclohexane, bis(isocyanatomethyl)norbornane, 3(4)-isocyanatomethyl-1-methylcyclohexyl isocyanate, 4,4'-bis(isocyanatocyclohexyl)methane or derivatives thereof with uretdione, isocyanurate, urethane, allophanate, biuret, iminooxadiazinedione and/or oxadiazinetrione structure with more than two NCO groups.

4. Layer composite according to any of Claims 1 to 3, **characterized in that** the isocyanate-reactive groups of the amine c) and/or of the amine h) comprise amino groups and optionally additionally hydroxyl groups.

5. Layer composite according to any of Claims 1 to 4, **characterized in that** the amine c) and/or the amine h) comprise/comprises one or more compounds selected from the group of hydrazine, ethylenediamine, 1,2-diaminopropane, 1,3-diaminopropane, 1,4-diaminobutane, 1,6-diaminohexane, isophoronediamine, isomer mixtures of 2,2,4- and 2,4,4-trimethylhexamethylenediamine, 2-methylpentamethylenediamine, diethylenetriamine, 1,3- and 1,4-xylylenediamine, α,α,α',α'-tetramethyl-1,3- and -1,4-xylylenediamine and 4,4'-diaminodicyclohexylmethane, dimethylethylenediamine, adipic dihydrazide, 1,4-bis(aminomethyl)cyclohexane, 4,4'-diamino-3,3'-dimethyldicyclohexylmethane, N-aminoethylethanolamine, ethanolamine, 3-aminopropanol, neopentanolamine, and diethanolamine.

6. Layer composite according to any of Claims 1 to 5, **characterized in that** the monofunctional polyoxyalkylene ether d) and/or the monofunctional polyoxyalkylene ether i) comprise/comprises ethylene oxide units and propylene oxide units.

7. Layer composite according to Claim 6, **characterized in that** the monofunctional polyoxyalkylene ether d) and/or the monofunctional polyoxyalkylene ether i) comprise/comprises at least 40 mol% of ethylene oxide units and not more than 60 mol% of propylene oxide units.

8. Layer composite according to any of Claims 1 to 7, **characterized in that** the average molar weight of the monofunctional polyoxyalkylene ether d) and/or of the monofunctional polyoxyalkylene ether i) is 500 g/mol to 5000 g/mol, preferably 1000 g/mol to 4000 g/mol, and more preferably 1000 to 3000 g/mol.

9. Layer composite according to any of Claims 1 to 8, **characterized in that** the isocyanate-reactive groups of the carboxylic acid e) are hydroxyl and/or amino groups.

10. Layer composite according to any of Claims 1 to 9, **characterized in that** the carboxylic acid e) comprises one or more compounds selected from the group of monohydroxycarboxylic acids, dihydroxycarboxylic acids, trihydroxycarboxylic acids, monoaminocarboxylic acids, diaminocarboxylic acids, and triaminocarboxylic acids.

11. Layer composite according to any of Claims 1 to 9, **characterized in that** the carboxylic acid e) comprises one or more compounds selected from the group of dimethylolpropionic acid, dimethylolbutyric acid, dimethylolacetic acid, dihydroxysuccinic acid, hydroxypivalic acid, hydroxyacetic acid, hydroxypropionic acid, 6-aminohexanoic acid, alanine, N-(2-aminoethyl)-ß-alanine, aminoundecanoic acid, 8-aminooctanoic acid, 5-aminopentanoic acid, 4-aminobutyric acid, aminobenzoic acid, 4-aminomethylcyclohexanecarboxylic acid, 2-aminohexanoic acid, 4-aminocyclohexanecarboxylic acid, 12-aminododecanoic acid, and 9-aminononanecarboxylic acid.

12. Layer composite according to any of Claims 1 to 11, **characterized in that** the adhesion promoter layer is applied to a substrate, preferably to a medical device, and more preferably to a catheter, a guidewire, an endoscope, a stent, a dilatation balloon, or to a blood filter.

13. Layer composite according to any of Claims 1 to 12, **characterized in that** the adhesion promoter layer has a thickness in the range from 1 to 200 µm, preferably from 1 to 150 µm, and more preferably from 1 to 100 µm.

14. Layer composite according to any of Claims 1 to 13, **characterized in that** the hydrophilic lubricity layer has a thickness in the range from 1 to 500 µm, preferably from 1 to 250 µm, and more preferably from 5 to 100 µm.

15. Method for producing a layer composite according to any of Claims 1 to 14, **characterized in that** the adhesion promoter layer is applied to a substrate and the hydrophilic lubricity layer is applied at least regionally to the adhesion promoter layer.

## Revendications

1. Composite à couches, comprenant une couche de promoteur d'adhérence et un revêtement de finition hydrophile lié à celle-ci, la couche de promoteur d'adhérence comprenant une polyuréthane-urée qui peut être obtenue par transformation au moins des composants suivants :
a) un macropolyol ;
b) un polyisocyanate ;
c) une amine présentant au moins deux groupes réactifs avec isocyanate ;
d) un polyoxyalkylène-éther monofonctionnel ; et
e) un acide carboxylique présentant au moins deux groupes réactifs avec isocyanate,
le revêtement de finition hydrophile comprenant une polyuréthane-urée qui peut être obtenue par transformation au moins des composants suivants :
f) un macropolyol ;
g) un polyisocyanate ;
h) une amine présentant au moins deux groupes réactifs avec isocyanate ; et
i) un polyoxyalkylène-éther monofonctionnel ;
et la polyuréthane-urée du revêtement de finition hydrophile ne présentant pas de groupes acide carboxylique.

2. Composite à couches selon la revendication 1, **caractérisé en ce que** le macropolyol a) et/ou le macropolyol f) comprend un ou plusieurs composés choisis dans le groupe formé par polyesterpolyol, polyétherpolyol, polycarbonatepolyol.

3. Composite à couches selon l'une quelconque des revendications 1 ou 2, **caractérisé en ce que** le polyisocyanate b) et/ou le polyisocyanate g) comprend un ou plusieurs composés choisis dans le groupe formé par le diisocyanate d'hexaméthylène, le diisocyanate de triméthylhexaméthylène, le 1,5-diisocyanate de 2-méthylpentane, le diisocyanate d'isophorone, le 1,3-bis(isocyanatométhyl)cyclohexane et/ou le 1,4-bis(isocyanatométhyl)cyclohexane, le bis(isocyanatométhyl)norbornane, l'isocyanate de 3(4)-isocyanatométhyl-1-méthylcyclohexyle, le 4,4'-bis(isocyanatocyclohexyl)-méthane ou leurs dérivés avec une structure uretdione, isocyanurate, uréthane, allophanate, biuret, iminooxadiazinedione et/ou oxadiazinetrione présentant plus de deux groupes NCO.

4. Composite à couches selon l'une quelconque des revendications 1 à 3, **caractérisé en ce que** les groupes réactifs avec isocyanate de l'amine c) et/ou de l'amine h) comprennent des groupes amino et le cas échéant en outre des groupes hydroxy.

5. Composite à couches selon l'une quelconque des revendications 1 à 4, **caractérisé en ce que** l'amine c) et/ou l'amine h) comprend/comprennent un ou plusieurs composés choisis dans le groupe formé par l'hydrazine, l'éthylènediamine, le 1,2-diaminopropane, le 1,3-diaminopropane, le 1,4-diaminobutane, le 1,6-diaminohexane, l'isophoronediamine, les mélanges des isomères de la 2,2,4-triméthylhexaméthylènediamine et de la 2,4,4-triméthylhexaméthylènediamine, la 2-méthylpentaméthylènediamine, la diéthylènetriamine, la 1,3-xylylènediamine et la 1,4-xylylènediamine, l'α,α,α',α'-tétraméthyl-1,3-xylylènediamine et l'α,α,α',α'-tétraméthyl-1,4-xylylènediamine et le 4,4'-diaminodicyclohexylméthane, la diméthyléthylènediamine, le dihydrazide de l'acide adipique, le 1,4-bis(aminométhyl)cyclohexane, le 4,4'-diamino-3,3'-diméthyldicyclohexylméthane, la N-aminoéthyléthanolamine, l'éthanolamine, le 3-aminopropanol, la néopentanolamine, la diéthanolamine.

6. Composite à couches selon l'une quelconque des revendications 1 à 5, **caractérisé en ce que** le polyoxyalkylène-éther monofonctionnel d) et/ou le polyoxyalkylène-éther monofonctionnel i) comprend/comprennent des unités d'oxyde d'éthylène et des unités d'oxyde de propylène.

7. Composite à couches selon la revendication 6, **caractérisé en ce que** le polyoxyalkylène-éther monofonctionnel d) et/ou le polyoxyalkylèneéther monofonctionnel i) comprend/comprennent au moins 40% en mole d'unités d'oxyde d'éthylène et au maximum 60% en mole d'unités d'oxyde de propylène.

8. Composite à couches selon l'une quelconque des revendications 1 à 7, **caractérisé en ce que** le poids moléculaire moyen du polyoxyalkylène-éther monofonctionnel d) et/ou du polyoxyalkylène-éther monofonctionnel i) est de 500 g/mole à 5000 g/mole, de préférence de 1000 g/mole à 4000 g/mole et de manière particulièrement préférée de 1000 à 3000 g/mole.

9. Composite à couches selon l'une quelconque des revendications 1 à 8, **caractérisé en ce que** les groupes réactifs avec isocyanate de l'acide carboxylique e) sont des groupes hydroxy et/ou amino.

10. Composite à couches selon l'une quelconque des revendications 1 à 9, **caractérisé en ce que** l'acide carboxylique e) comprend un ou plusieurs composés choisis dans le groupe formé par les acides monohydroxycarboxyliques, dihydroxycarboxyliques, trihydroxycarboxyliques, monoaminocarboxyliques, diaminocarboxyliques, triaminocarboxyliques.

11. Composite à couches selon l'une quelconque des revendications 1 à 9, **caractérisé en ce que** l'acide carboxylique e) comprend un ou plusieurs composés choisis dans le groupe formé par l'acide diméthylolpropionique, l'acide diméthylolbutyrique, l'acide diméthylolacétique, l'acide dihydroxysuccinique, l'acide hydroxypivalique, l'acide hydroxyacétique, l'acide hydroxypropionique, l'acide 6-aminohexanoïque, l'alanine, la N-(2-aminoéthyl)-ß-alanine, l'acide aminoundécanoïque, l'acide 8-aminooctanoïque, l'acide 5-aminopentanoïque, l'acide 4-aminobutyrique, l'acide aminobenzoïque, l'acide 4-aminométhylcyclohexanecarboxylique, l'acide 2-aminohexanoïque, l'acide 4-aminocyclohexanecarboxylique, l'acide 12-aminododécanoïque, l'acide 9-aminononacarboxylique.

12. Composite à couches selon l'une quelconque des revendications 1 à 11, **caractérisé en ce que** la couche de promoteur d'adhérence est appliquée sur un substrat, de préférence sur un appareil médical et de manière particulièrement préférée sur un cathéter, un filguide, un endoscope, une endoprothèse, un ballonnet de dilatation ou un filtre sanguin.

13. Composite à couches selon l'une quelconque des revendications 1 à 12, **caractérisé en ce que** la couche de promoteur d'adhérence présente une épaisseur dans la plage de 1 à 200 µm, de préférence de 1 à 150 µm et de manière particulièrement préférée de 1 à 100 µm.

14. Composite à couches selon l'une quelconque des revendications 1 à 13, **caractérisé en ce que** la couche hydrophile de glissement présente une épaisseur dans la plage de 1 à 500 µm, de préférence de 1 à 250 µm et de manière particulièrement préférée de 5 à 100 µm.

15. Procédé pour la fabrication d'un composite à couches selon l'une quelconque des revendications 1 à 14, **caractérisé en ce que** la couche de promoteur d'adhérence est appliquée sur un substrat et la couche hydrophile de glissement est appliquée au moins par zones sur la couche de promoteur d'adhérence.
